# EUROPEAN PATENT SPECIFICATION

(11) **EP 1 656 455 B1**
(45) Date of publication and mention of the grant of the patent: **19.09.2012**
(21) Application number: 04764055.2
(22) Date of filing: 12.08.2004
(51) Int. Cl.: C12P 21/00, C07K 14/56

(54) **PROCESS FOR THE PURIFICATION OF RECOMBINANT POLYPEPTIDES**
VERFAHREN ZUR REINIGUNG REKOMBINANTER POLYPEPTIDE
PROCÉDÉ DE PURIFICATION DES POLYPEPTIDES RECOMBINANTS

(30) Priority: 13.08.2003 US 494915 P
(43) Date of publication of application: 17.05.2006
(73) Proprietor: SANDOZ AG, 4056 Basel (CH)
(72) Inventor: STEMPFER, Günter, A-6233 Kramsach (AT); ALLIGER, Peter, A-6330 Kufstein (AT); PALMA, Norbert, A-6252 Breitenbach a. Inn (AT)
(74) Representative: Kröger, Bernd
(86) International application number: PCT/EP2004/009055
(87) International publication number: WO 2005/017174

(56) References cited:
- EP-B1- 0 679 718
- WO-A-86/04067
- WO-A-89/00201
- WO-A-03/004599
- US-A- 6 005 075
- KIM DONG CHUNG ET AL: "Purification of recombinant human alpha-2a interferon without using monoclonal antibodies." JOURNAL OF MICROBIOLOGY AND BIOTECHNOLOGY, vol. 12, no. 6, December 2002 (2002-12), pages 916-920, XP009078402 ISSN: 1017-7825
- HART R A ET AL: "LARGE SCALE, IN SITU ISOLATION OF PERIPLASMIC IGF-I FROM E. COLI" BIO/TECHNOLOGY, NATURE PUBLISHING CO. NEW YORK, US, vol. 12, November 1994 (1994-11), pages 1113-1117, XP001153305 ISSN: 0733-222X
- HART R A ET AL: "LARGE SCALE, IN SITU ISOLATION OF PERIPLASMIC IGF-I FROM E. COLI" BIO/TECHNOLOGY, NATURE PUBLISHING CO. NEW YORK, US, vol. 12, November 1994 (1994-11), pages 1113-1117, XP001153305 ISSN: 0733-222X
- FRENCH C ET AL: "DEVELOPMENT OF A SIMPLE METHOD FOR THE RECOVERY OF RECOMBINANT PROTEINS FROM THE ESCHERICHIA COLI PERIPLASM" ENZYME AND MICROBIAL TECHNOLOGY, STONEHAM, MA, US, vol. 19, no. 5, 1996, pages 332-338, XP000886796 ISSN: 0141-0229
- DALMORA S ET AL: "Analysis of recombinant human growth hormone directly in osmotic shock fluids" JOURNAL OF CHROMATOGRAPHY A, ELSEVIER SCIENCE, NL, vol. 782, no. 2, 10 October 1997 (1997-10-10), pages 199-210, XP004096275 ISSN: 0021-9673

## Description

This invention is concerned with a method for preparation of a recombinant polypeptide of interest, which polypeptide upon expression has been secreted into the periplasm of a transformed host cell.

In particular, this invention is concerned with methods of preparing and purifying a recombinant human interferon alpha 2.

Polypeptides or proteins, like interferons of the group of interferon alpha 2, may be produced by recombinant DNA technology using bacterial cells (e.g. Escherichia coli) as hosts. Thus, bacterial cells may be transformed with plasmid DNA encoding said polypeptide. The bacteria are thereby enabled to express quantities of the polypeptide in either the cytoplasm or the periplasm. As the bacteria can be grown in large amounts using large-scale fermentation processes, it is possible to produce large quantities of the polypeptide in this way.

Whereas recombinant techniques can be employed to produce high yields of a crude polypeptide of interest, the isolation and purification of the polypeptide is not a simple matter. In a typical isolation procedure, a fermentation broth as neutralised, for example by acidification or heating. Thereafter, the bacterial cells are removed to leave a liquid supernatant, containing unwanted soluble by-products, which is discarded. The resultant bacterial cell mass is re-suspended in an appropriate medium, e.g. a suitable buffer and the cells are disrupted to extract and isolate the crude interferon. This laborious procedure is carried out in order to separate the polypeptide of interest from as much fermentation by-products and other contaminants as possible to ensure that subsequent purification steps (involving chromatographic separation) proceed in as an efficient manner as possible.

The laborious nature of this prior art procedure may lead to lower yields of extracted polypeptide of interest and higher production costs. Accordingly, there remains a need for a process that enables the preparation of a recombinant polypeptide of interest from bacterial cells in a high yielding and cost-effective manner.

Examples of prior art procedures are as follows:
Hart et al. describe in situ isolation of periplasmic IGF-I from E. coli. IGF-I was solubilized by chaotropes and reductants under alkaline conditions (Hart, R.A., Lester, P.M., Reifsnyder, D.H., et al. (1994). Bio/Technology, vol. 12, pp. 1113-1117). An aqueous two-phase system was designed to improve subsequent centrifugation and recovery yield.
Dalmora et al. describe the use of osmotic shock in a small-scale analytical procedure (Dalmora et al. (1997). Journal of Chromatography A, vol. 782, pp. 199-210). Before applying an osmotic shock, the cells were separated from the fermentation broth by centrifugation.

In the context of the present invention it has surprisingly been found that efficient extraction and isolation of a recombinant polypeptide of interest, for example of a recombinant interferon alpha 2, from a host cell comprising a periplasm, like a Gram-negative bacterial cell, is possible by directly performing an osmotic shock on the host cells comprising an expressed recombinant polypeptide of interest in their periplasm, thereby omitting the aforementioned separation and re-suspension steps. Upon performance of such a process the outer cell membrane of the host cell is sufficiently disrupted as to release the contents of its periplasm into the fermentation medium.

Thereby, the release of unwanted cytoplasmic material, e.g. host-cell proteins and DNA in the cell debris fraction, can be avoided. Surprisingly, subsequent chromatographic purification is not compromised, but readily leads to superior yields and/or purity of the recombinant polypeptide to be isolated.

In the context of the present invention it has furthermore been found that a unique sequence of chromatographic steps, in particular if combined with the extraction / disruption process mentioned above, leads to superior yields and/or purity of a recombinant interferon alpha 2.

Accordingly, in one aspect of the present invention, there is provided a process for the preparation of a recombinant polypeptide of interest, comprising
(i) fermentation of a prokaryotic host cell comprising a periplasm and being transformed with a recombinant expression system capable of bringing about secretion of a polypeptide of interest into the periplasm of said host cell, wherein said fermentation is performed in a fermentation medium under conditions such that the polypeptide of interest is secreted into the periplasm of the host cell,
(ii) extraction of the polypeptide of interest from the periplasm by applying an osmotic shock to the host cells contained in the fermentation medium,
wherein said osmotic shock is performed by adding sucrose directly to the fermentation medium and subsequent dilution with H₂O such that the outer cell membrane of the host cell is sufficiently disrupted as to release the contents of its periplasm into the fermentation medium.

Suitable recombinant periplasmic expression systems, in particular expression vectors, and corresponding prokaryotic host cells as well as appropriate fermentations methods are well known in the art. Suitable examples are described below in the Examples section.

In a preferred embodiment thereof said osmotic shock is performed by adding an agent directly to the fermentation medium, wherein said agent is capable of creating after dilution with H₂O an osmotic pressure leading to disruption of the outer cell membrane of the host cell, and subsequent dilution with H₂O.

The agent is of sucrose. Optionally, a complex forming component, like EDTA, may additionally be added to the fermentation medium.

The agent is present in such a concentration as, upon dilution of the fermentation medium with H₂O, to bring about an osmotic shock which leads to disruption of outer cell membrane of the host cell with subsequent release of the expressed polypeptide of interest.

In particular, in a further preferred embodiment of the present invention, the concentration of the sucrose in the fermentation medium when starting the dilution is about 20% weight/volume. Preferably, the dilution factor of the sucrose-containing fermentation broth with H₂O is at least 3 times.

In the context of the present invention, a preferred prokaryotic host cell comprising a periplasm is a Gram-negative bacterium. Preferably, the said Gram-negative bacterium is selected from the group consisting of Escherichia coli, Pseudomonas sp., Enterobacter sp., Campylobacter sp. and Vitreoscilla sp. In a most preferred embodiment of the present invention, the host cell is E. coli.

Subsequent to the cell disruption step, the fermentation broth, being a crude preparation of the recombinant polypeptide of interest, may be subjected to a separation step, e.g. high speed centrifugation, in order that cellular debris and other particulate matter can be separated from the extract containing the polypeptide of interest. To assist in the separation of particulate matter from the extract it is preferred to add to the fermentation broth prior to the separation step, a suitable precipitating agent. In the context of the present invention it has been found that polyethyleneimine is a particularly good precipitating agent for this step. The polyethylenelmine is preferably employed at a concentration of about 0.05% and in a medium which is pH adjusted to about 7.5, e.g. 7.3 to 7.7.

The process of the present invention can be utilized in the production of a large variety of polypeptides of interest. In particular, in accordance with the present invention, the polypeptide of interest can be selected from the group consisting of an interferon, an interleukin, a growth hormone, a growth factor, a cytokine, an enzyme, an enzyme inhibitor, an antibody and an antibody fragment, and the like, for example interferon alpha 2A, interferon alpha 2B, interleukin-3, interleukin-6, human growth hormone, insulin, granulocyte-colony stimulating factor, granulocyte macrophage-colony stimulating factor, macrophage-colony stimulating factor, interferon beta 1, bovine somatropin, porcine somatropin, interleukin-11, interleukin-2, a Fab-fragment, and small peptides such as calcitonin, parathyroid hormone (PTH), or a glucagon. Preferably, within the scope of the present invention, the polypeptide of interest is a recombinant human interferon 2, in particular human interferon alpha 2A or human interferon alpha 2B, the latter being particularly preferred to be the polypeptide of interest.

The extract comprising the polypeptide of interest may contain a number of impurities, for example host-cell proteins and host-cell DNA which have to be removed before the interferon can be formulated into a finished dosage form. Typically, the polypeptide is purified by precipitation and chromatographic separation techniques, which are known per se. For example, the polypeptide may be purified using multi-step chromatographic separation.

However, depending on the nature of the polypeptide of interest, the process is complicated by the need for significant dialysis and/or concentration steps interposed between the chromatographic steps. These extra steps are laborious and may lead to lower yields and higher production costs.

In the context of the present invention it has been found that a multi-step chromatographic purification of a crude preparation, in particular prepared as described above, of recombinant interferon alpha 2, may be carried out without any dialysis or concentration steps by the judicious selection and ordering of certain chromatographic steps.

Therefore, another aspect of the present invention relates to a process for the preparation of a recombinant interferon alpha 2, comprising
(a) obtaining a crude preparation of a recombinant interferon alpha 2,
(b) applying the crude preparation to a multi-step chromatography comprising the following steps in sequence:
   (i) cation exchange chromatography,
   (ii) anion exchange chromatography,
   (iii) hydrophobic interaction chromatography,
   (iv) cation exchange chromatography,
   (v) size exclusion chromatography.

In a preferred embodiment thereof, the crude preparation of the recombinant interferon alpha 2 is obtained by a process comprising
(a) fermentation of a prokaryotic host cell comprising a periplasm and being transformed with a recombinant expression system capable of bringing about secretion of a recombinant interferon alpha 2 into the periplasm of said host cell, wherein said fermentation is performed in a fermentation medium under conditions such that the recombinant interferon alpha 2 is secreted into the periplasm,
(b) extraction of the recombinant interferon alpha 2 from the periplasm by applying an osmotic shock to the host cells contained in the fermentation medium.

As mentioned herein, suitable recombinant periplasmic expression systems, in particular expression vectors, and corresponding prokaryotic host cells as well as appropriate fermentations methods are well known in the art. Suitable examples are described below in the Examples section.

Preferably and as mentioned above, said osmotic shock is performed by adding an agent directly to the fermentation medium, wherein said agent is capable of creating after dilution with H₂O an osmotic pressure leading to disruption of the outer cell membrane of the host cell, and subsequent dilution with H₂O.

The agent is sucrose. Optionally, a complex forming component, like EDTA, may additionally be added to the fermentation medium.

As mentioned herein, the agent is present in such a concentration as, upon dilution of the fermentation medium with H₂O, to bring about an osmotic shock which leads to disruption of outer cell membrane of the host cell with subsequent release of the expressed polypeptide of interest.

Preferably, the concentration of the sucrose in the fermentation medium when starting the dilution is about 20% weight/volume. In a preferred embodiment thereof, the dilution factor for the sucrose-containing fermentation broth with H₂O is at least 3 times.

As mentioned herein, a preferred prokaryotic host cell is a Gram-negative bacterium, which preferably is selected from the group consisting of Escherichia coli, Pseudomonas sp., Enterobacter sp., Campylobacter sp. and Vitreoscilla sp, E. coli being particularly preferred.

Said interferon alpha 2 preferably is selected from the group consisting of interferon alpha 2A and interferon alpha 2B. In a most preferred embodiment, said interferon alpha 2 is interferon alpha 2B.

In the extraction method hereinabove described it is possible to obtain a clear crude extract containing the recombinant interferon alpha 2. The polypeptide-containing extract will have a high content of the interferon alpha 2 in highly purified form.

In carrying out the cation exchange chromatography (CEX) step, the pH of the crude interferon alpha 2-containing extract obtained from the extraction step may be adjusted to a pH of about 4.8 to 5.5 and may optionally be passed through a filter system (e.g. 0.3 micrometre filter system). The treated extract is thereafter eluted on a CEX column. Any cation-exchange column known in the art may be useful for separating the extracted interferon alpha 2 from impurities. Preferably however, the column is packed with S ceramic Hyper D F. The equilibration eluent is preferably sodium acetate (20mM) + NaCl (70mM) at a pH of 5.0. The interferon is run on the column preferably using a step gradient at 175mM NaCl.

The pH of the desired interferon alpha 2-containing fraction eluted from the CEX column may be adjusted to about 7.3. to 7.7 with an appropriate alkaline material, e.g. sodium hydroxide, and the conductivity of the fraction may be adjusted to about 3.5 to 4.5 mS/cm by dilution using purified water.

Thereafter, the fraction is fed onto an anion-exchange column to effect the process of step ii). Any anion exchange column known in the art may be useful for separating the extracted interferon from impurities. Preferably however, the column is packed with Ceramic Q HyperD F resin. The equilibration is preferably 20mM Tris-HCl at pH 7. and thus at high flow rates, e.g. 4 - 8 cm/min. The desired interferon fraction may be eluted after washing the column with equilibration buffer by adding a suitable ionic solute, e.g. sodium chloride at a concentration of up to 1000 mM, preferably 150mM. The temperature at which the separation is run is preferably from 10°C to 15°C.

This anion exchange step is highly efficient and the purity of the interferon in the interferon fraction resultant from step ii) may be greater than 40% as determined by reverse-phase high performance liquid chromatography.

After the completion of step ii) the interferon fraction may still be contaminated with host-cell proteins. Accordingly, step iii) in the purification process is a Hydrophobic Interaction Chromatography (HIC) step and is adapted to remove, *inter alia* substantial amounts of these proteins. The step is carried out on a column packed with a suitable resin for this purpose. Preferably the resin is 15PHE (Pharmacia). In a preferred step iii) the interferon fraction to be eluted on the column is first diluted (1:1) with a sodium sulphate solution to a concentration of 0.5M sodium sulphate. Thereafter, the fraction is pH adjusted to about 7.3 to 7.7 with a suitable acid or base, e.g. NaOH or HCl. This pH adjusted solution is then added to the HIC column. After a washing step, the interferon is eluted with a linear sodium sulphate gradient, preferably 800 to 0mM sodium sulphate. Fractions are collected and pooled that have a purity of greater than or equal to 93 area % interferon and no single impurity having greater than or equal to 3 area % according to IPC reversed-phase HPLC. The pooled fractions may be used immediately in the next step.

Step iv) is a further cation exchange chromatography (CEX) step which serves to remove residual DNA and residual host-cell proteins which may have been carried over from previous steps.

A CEX column is packed with a suitable packing material, e.g. Toyopearl SP-650 S (TosoHaas). In a preferred step iv) the fraction obtained from step iii) may be diluted with purified water to a final conductivity of about 7.5 to 8.5 mS/cm, adjusted to a pH of 4.3 to 4.7 with, for example with 99-100% acetic acid, and applied to the column. The column is washed and thereafter interferon alpha 2 is eluted during a linear sodium chloride gradient (0-300mM NaCl) at about 250mM NaCl. Eluted fractions having a purity of greater than or equal to 95 area% interferon main peak and no single impurity greater than or equal to 3 area % as measured by IPC reversed-phase HPLC may be collected and processed immediately in the next purification step.

Step v) is a size exclusion chromatography step which is employed to remove dimers and any other aggregates and, where applicable, also to perform a buffer change which may be necessary before the interferon product can be formulated into a finished dosage form.

Any column and packing material suitable for gel filtration may be employed for this final step. Preferably the packing material employed is Superdex 75pg. The packing material is chosen for its good resolution capabilities even at relatively high load volumes of, for example about 5 to 15%. Preferably the column is equilibrated with an equilibration buffer before being loaded with the interferon fraction from the previous step iv). Thereafter the interferon fraction is eluted off the column using a suitable buffer which preferably consists of 25 mM sodium phosphate, 130 mM sodium chloride and 0.3 mM EDTA at a pH of 7.1 - 7.7 to provide a final bulk solution containing the interferon alpha 2 product.

The process as hereinabove described constitutes an efficient process of obtaining an interferon alpha 2 product which is applicable on an industrial scale. It is possible to obtain yields of the interferon product exceeding 100 mg per liter of fermentation broth.

The interferon alpha 2 polypeptide of the present invention may be formulated into finished dosage forms suitable for administering to humans. Formulations containing interferon alpha 2 products of the present invention may be formulated as injectable formulations. Injectable formulations may be provided as lyophilised products that should be reconstituted with water before administration. Alternatively, injectable formulations may be provided as injectable for solutions as single or multidose preparations. Injectable formulations may additionally comprise excipients commonly known in the art.

Such formulations are useful in the treatment of Hepatitis C. The dosage may depend on the various factors such as the method of administration, age and/or individual condition.

The following examples serve to illustrate the present invention, without in any way limiting the scope thereof. Subject-matter disclosed in the examples relates to preferred embodiments of the present invention.

### Examples

### Example 1: Construction of a host cell strain for production of recombinant human interferon alpha 2B (rhIFNα2B)

### 1.1 General considerations

The polypeptide rhIFNα2b (recombinant human Interferon-α2b) is produced in the *Escherichia coli* K-12 strain W3110 transformed with a plasmid containing an optimized synthetic gene coding for rhIFNα2b. rhIFNα2b is produced under the control of the promoter and Ribosome Binding Site (RBS) of the glutaryl 7-ACA acylase gene (*gac*) from *Pseudomonas diminuta* CCM 3987 by fermentation of recombinant *E. coli* K-12. rhIFNα2b is expressed as an N-terminal fusion protein with the signal sequence from the same (*gac*) gene, directing the protein to the periplasm with concurrent processing (cleaving off) of the signal sequence. The fermentation process therefore directly yields mature rhIFNα2b with a primary sequence identical to that of naturally occurring human Interferon alpha 2b. The expression plasmid is designated pMG414, the production strain W3110[pMG414].

### 1.2 Construction of expression vector pMG414

pUC19 serves as the starting point for the construction of the vector plasmid. pUC19 is a frequently used and thoroughly characterized high copy plasmid. It contains a highly efficient origin of replication and an ampicillin resistance (*amp or bla*) gene (Yanisch-Perron *et al.,* 1985; Vieira and Messing, 1982; GenBank accession numbers L09137 and X02514). Even though pUC19 is frequently used for the construction of expression plasmids, the amp gene may not be an ideal selectable marker for industrial purposes. For this reason the promoter and the coding region of the *amp* gene are removed and replaced by the promoter and the coding region of the tetracycline resistance gene (*tet*) from the well known safety plasmid pBR322 (Bolivar et al., 1977a, 1977b, 1978; review: Balbás et al., 1986; GenBank accession numbers J01749, K00005, L08654, M10283, M10286, M10356, M10784, M10785, M10786, M33694, V01119). This cloning work is performed with the help of high fidelity PCR techniques.

To achieve this, the fragment spanning bps 1743 to 679 of pUC19 is amplified using high fidelity PCR (Pwo DNA Polymerase system from Roche Biochemicals) and the following 5'-phosphorylated oligonucleotides:
Oligo 235: 5'- Phosphate - TAACTGTCAG ACCAAGTTTA CTC -3' (SEQ ID NO 1)
Oligo 236: 5'- Phosphate - GCGTTTCGGT GATGACGGTG -3' (SEQ ID NO 2)

The resulting PCR fragment is 1624 bps in length and contains the complete pUC19 backbone lacking the *amp* promoter and coding sequence, but including the stop codon and transcription terminator from the *amp* gene.

As mentioned above, the *tet* promoter and coding sequence (excluding the stop codon) is amplified from pBR322. Again, high fidelity PCR was used to amplify bps 4 to 1273 of pBR322. The following 5'-phosphorylated oligonucleotides were used for this amplification:
Oligo 237: 5'- Phosphate - TCATGTTTGA CAGCTTATCA TCG -3' (SEQ ID NO 3)
Oligo 238: 5'- Phosphate - GGTCGAGGTG GCCCGGCTC -3' (SEQ ID NO 4)

The resulting PCR fragment is 1270 bps in length. The two PCR fragments are purified by preparative agarose gel electrophoresis and ligated using T4 DNA Ligase (Rapid DNA Ligation Kit, Roche Biochemicals). The ligated DNA is purified and electroporated into *Escherichia coli* K-12 DH10B (Life Technologies ElectroMAX DH10B electrocompetent cells, genotype: F⁻ *mcr*A Δ(*mrr-hsd*RMS-*mcr*BS) φ80d*lac*ZΔM15 Δ*lac*X74 deoR recA1 *end*A1 *ara*D139 Δ(*ara*, *leu*)7697 *gal*U *ga*/K λ⁻ *rps*L *nup*G). Transformed cells are plated on to LB agar 15 mg/L tetracycline and 3 g/L glucose. Liquid cultures are grown in LB broth containing 15 mg/L tetracycline and 3 g/L glucose and plasmid DNA is isolated from these cultures using standard miniprep methods. Plasmid DNAs are analyzed by restriction analysis for correct integration of the *tet* fragment into the pUC19 backbone. Since integration of the fragment was unspecific with respect to orientation, only about 50% of all insert containing clone had the fragment inserted in the correct orientation, i.e. the *tet* gene running in the same direction as the the amp gene in pUC19. A larger amount of DNA is isolated from liquid cultures of a few clones and subjected to more detailed restriction analyses. Of those clones showing correct restriciton patterns, one is selected for further cloning work.

The respective plasmid was designated pMG402. It is identical to pUC19 in all features and functions but for the fact that it must be grown on/in tetracycline-containing media instead of ampicillin-containing media. This way a *tet* resistant high copy vector suitable for industrial purposes is generated.

### Features of plasmid pMG402:

bps 1954-680: pUC19 backbone (= pUC19 lacking the amp promoter and structural gene)
bps 681-1953: *tet* promoter and structural gene from pBR322

rhIFNα2b is expressed as an N-terminal fusion with the signal sequence of glutaryl 7-ACA acylase from *Pseudomonas diminuta* CCM 3987 (gac1ss = SEQ ID NO 5) directing the protein to the periplasm with concurrent processing (cleaving off) of the signal sequence by the host cell's signal peptidase apparatus.

### Amino acid sequence of gac1ss (27 aa): MLRVLHRAAS ALVMATVIGL APAVAFA

In the 3' region of the coding sequence of the gac1ss a Sac II restriction endonuclease site is introduced via the 3' PCR primer creating a silent mutation (amino acid sequence unchanged). This Sac II site allows fusion of the gac1ss coding region with the rhIFNα2b gene.

The structural gene for rhIFNα2b is synthesized chemically. It differs from the natural human cDNA sequence in 48 of 165 codons and is designed to eliminate any weak and error prone codons.

In the following table, codon changes are indicated. In the table, "Natural codon" refers to the cDNA sequence published by Streuli et al., 1980 (GenBank Accession Number V00548). The amino acid numbers refer to mature hIFNα2b (starting with Cys 1). The amino acid sequence to be encoded by the synthetic gene is taken from the SwissProt Database, Accession number P01563/P01564 (amino acids 24 to 188).

| **Exchange no.** | **Amino acid** | **Natural codon** | **Synthetic codon** | **Exchange no.** | **Amino acid** | **Natural codon** | **Synthetic codon** |
|---|---|---|---|---|---|---|---|
| **1** | Cys 1 | TGT | TGC | **25** | Leu 80 | CTC | CTG |
| **2** | Pro 4 | CCT | CCG | **26** | Leu 81 | CTA | CTT |
| **3** | Arg 12 | AGG | CGG | **27** | Leu 88 | CTC | CTG |
| **4** | Arg 13 | AGG | CGA | **28** | Asn 93 | AAT | AAC |
| **5** | Leu 17 | CTC | CTT | **29** | Cys 98 | TGT | TGC |
| **6** | Arg 22 | AGG | CGG | **30** | Ile 100 | ATA | ATC |
| **7** | Arg 23 | AGA | CGA | **31** | Gly 102 | GGG | GGT |
| **8** | Ser 28 | TCC | TCT | **32** | Gly 104 | GGG | GGT |
| **9** | Leu 30 | TTG | TTA | **33** | Thr 106 | ACA | ACT |
| **10** | Asp 32 | GAC | GAT | **34** | Pro 109 | CCC | CCG |
| **11** | Arg 33 | AGA | CGA | **35** | Ser 115 | TCC | TCT |
| **12** | Phe 36 | TTT | TTC | **36** | Arg 120 | AGG | CGA |
| **13** | Gly 37 | GGA | GGT | **37** | Arg 125 | AGA | CGG |
| **14** | Phe 38 | TTT | TTC | **38** | Leu 128 | CTC | CTG |
| **15** | Pro 39 | CCC | CCG | **39** | Pro 137 | CCT | CCG |
| **16** | Phe 43 | TTT | TTC | **40** | Cys 138 | TGT | TGC |
| **17** | Gly 44 | GGC | GGT | **41** | Arg 144 | AGA | CGA |
| **18** | Pro 54 | CCT | CCG | **42** | Arg 149 | AGA | CGG |
| **19** | Val 55 | GTC | GTA | **43** | Phe 151 | TTT | TTC |
| **20** | Leu 56 | CTC | TTG | **44** | Ser 154 | TCA | TCT |
| **21** | Asn 65 | AAT | AAC | **45** | Thr 155 | ACA | ACC |
| **22** | Leu 66 | CTC | CTG | **46** | Ser 160 | AGT | TCT |
| **23** | Thr 69 | ACA | ACT | **47** | Arg 162 | AGA | CGA |
| **24** | Ser 72 | TCA | TCT | **48** | Ser 163 | AGT | AGC |

The resulting gene allows efficient and precise transcription and translation of rhIFNα2b in *Escherichia coli.* Since the gene is designed for expression in a bacterial system it does not contain any untranslated sequences (introns etc.).

The structural gene is chemically synthesized. In brief, overlapping complementary oligonucleotides about 30 to 50 nucleotides in length are synthesized in a way to cover both strands of the structural gene sequence without any gaps. The oligonucleotides are hybridized to each other and ligated using T4 DNA Ligase. The reaction product is cut with restriction endonucleases and cloned into the pUC18 vector. The resulting plasmid is sequenced and shows the correct sequence.

The synthetic gene on this plasmid does not contain the *gac* signal sequence. This part of the coding region is introduced via the gac fragment containing promoter, RBS and signal sequence and fused to the rhIFNα2b structural gene.

The *gac* fragment is generated by chemical synthesis. For example, overlapping complementary oligonucleotides about 30 to 50 nucleotides in length are synthesized in a way to cover the full length of both strands of the *gac* fragment (including the restriction endonuclease recognition sites on both sides plus a minimum of 6 additional basepairs to allow efficient cleavage) without any gaps. The oligonucleotides are then hybridized to each other (e.g. by heating and subsequent cooling) and ligated using T4 DNA Ligase. The reaction product is then cut with the respective restriction endonucleases (*Xba* I and *EcoR* I) and cloned into the pMG402 vector (see below).

In the alternative, the *gac* fragment containing promoter, RBS and signal sequence can be amplified from a plasmid comprising such elements like plasmid pKS55, which construction is described in CS patent No. 278,515. The gac gene cloned therein has been derived from a strain of Pseudomonas diminuta (CCM 3987). Amplification is carried out using a high fidelity PCR system. The restriction endonuclease sites needed for cloning are introduced via the following PCR primers.

### Primers:

1. 5'-Phosphate - GGGGGGTCTAGACCAACAACATCTTCAACGTCTACC -3' (SEQ ID NO 6)
2. 5'-Phosphate - CC CCC CGA ATT CAC TAG TAC GCG TCT CTC TCC -3' (SEQ ID NO 7)

There will be no difference in performance between a *gac* fragment generated via high fidelity PCR amplification and a *gac* fragment generated by chemical synthesis.

The thus created gac fragment has the following nucleotide sequence (SEQ ID NO 8):

5'-GGGGGGTCTAGACCAACAACATCTTCAACGTCTACCCGACCAAGATTCAGGAGCCGTCGG
CCGACCTGGGCAATGGGATGTACAGCGGGCTTGCGCCGTTCGGCTTCACCGGCGGATCCT
GGTTCGTACGCGCCGCCTACAAGTGGTGATCTAGGGGAACGTTCCGGGGGCGTCGCTGCA
ACGGCGTCTCCGGATCTGGGTGAGAGGGGAAATCCATGCTGAGAGTTCTGCACCGGGCGG
CGTCCGCCTTGGTTATGGCGACTGTGATCGGCCTTGCGCCCGCGGAGAGAGACGCGTACT
AGTGAATTCGGGGGG-3'

The *gac* fragment (either synthetic or created via PCR) and the vector plasmid pMG402 are ligated using the *Xba* I and *EcoR* I sites. This way the expression vector pMG412 is generated.

The expression vector, pMG412, contains codons 1 - 23 + the first nucleotide of codon 24 of the gac signal sequence. Into codons 22-24 the Sac II site is introduced by silent mutation. Anything downstream of the Sac II site in pMG412 is primer or vector sequence.

The last two nt of codon 24 + codons 25-27 are introduced by the forward PCR primer for the target structural gene (rhIFNα2B, see above). Such a primer therefore contains the following elements:
Cutting overhang (e.g. 6 nucleotides) - Sac II site - tc gcc ttt gcg (SEQ ID NO 9)- hybridizing region corresponding to the 5' end of the "mature" target gene.

In particular, a suitable primer has the following nucleotide sequence (SEQ ID NO 10):
TT GCG CCC GCG GTC GCC TTT GCG - hybridizing region (Sac II underlined)

The last amino acids (24-27) of the gac signal sequence are V A F A (SEQ ID NO 11).

From the plasmid construct described above the rhIFNα2b gene is amplified using a high fidelity PCR system. The 5' PCR primer contains the Sac II site for fusing the gene with the *gac* fragment plus the last four codons of the gac signal sequence. The 3' primer contains the TAA (ochre) stop codon and the *Mlu* I site for cloning. The amplification of the Interferon alpha structural gene generates the following fragment (SEQ ID NO 12):

GGGGGGCCGCGGTCGCCTTTGCGTGCGATCTGCCGCAAACCCACAGCCTGGGTAGCCGGC
GAACCTTGATGCTTCTGGCACAGATGCGGCGAATCTCTCTTTTCTCTTGCTTAAAGGATC
GACATGACTTCGGTTTCCCGCAGGAGGAGTTCGGTAACCAGTTCCAAAAGGCTGAAACCA
TCCCGGTATTGCATGAGATGATCCAGCAGATCTTCAACCTGTTCAGCACTAAGGACTCTT
CTGCTGCTTGGGATGAGACCCTGCTTGACAAATTCTACACTGAACTGTACCAGCAGCTGA
ACGACCTGGAAGCCTGCGTGATCCAGGGTGTGGGTGTGACTGAGACTCCGCTGATGAAGG
AGGACTCTATTCTGGCTGTGCGAAAATACTTCCAACGGATCACTCTGTATCTGAAAGAGA
AGAAATACAGCCCGTGCGCCTGGGAGGTTGTCCGAGCAGAAATCATGCGGTCTTTCTCTT
TGTCTACCAACTTGCAAGAATCTTTACGAAGCAAGGAATAATACGCGTGAATTCGGGGGG

This rhIFNα2b PCR fragment and pMG412 are ligated using the *Sac* II and *Mlu* I sites. This way the final production/expression plasmid pMG414 was generated. Both strands of pMG414 are sequenced and show no differences to the expected sequence.

Features of plasmid pMG414 (total size 3668 bps):

| | |
|---|---|
| bps 2728-256: | pUC19 backbone, part 1 |
| bps 257-546: | *gac* fragment (promoter, RBS, signal sequence) |
| bps 547-1044: | synthetic rhIFNα2b gene (including TAA stop) |
| bps 1045-1454: | cloning sites + pUC19 backbone, part 2 |
| bps 1455-2727: | *tet* gene from pBR322 (promoter/RBS 1455-1536, coding sequence including TAA stop 1537-2727) |

Thereby, the *gac* fragment containing promoter, RBS and signal sequence is fused to the rhIFNα2b structural gene - using a restriction endonuclease site at the 3' end of the gac fragment introduced by a PCR primer. The same site is fused to the 5' end of the rhIFNα2b structural gene, also by the way of a PCR primer. So after cloning both elements (*gac* fragment and rhIFNα2b structural gene) into the basic vector a gene encoding a gac1ss-rhIFNα2b fusion protein is generated. Of its total 192 codons (576 nucleotides) the first 27 encode the gac signal sequence not present in the final protein and amino acids 28 to 192 encode mature rhIFNα2b (165 amino acids, cysteine 1 to glutamic acid 165)

The nucleotide sequence of the expression cassette used in the rhIFNα2b expression plasmid pMG414 (807 bps) (see below) and amino acid sequence of the gac1ss-rhIFNα2b fusion protein is shown as follows (SEQ ID NO 13):

5'-TCTAGACCAACAACATCTTCAACGTCTACCCGACCAAGATTCAGGAGCCGTCGGCCGACC
TGGGCAATGGGATGTACAGCGGGCTTGCGCCGTTCGGCTTCACCGGCGGATCCTGGTTCG
TACGCGCCGCCTACAAGTGGTGATCTAGGGGAACGTTCCGGGGGCGTCGCTGCAACGGCG
TCTCCGGATCTGGGTGAGAGGGGAAATCC

ATG CTG AGA GTT CTG CAC CGG GCG GCG TCC GCC TTG GTT ATG GCG
M L R V L H R A A S A L V M A

ACT GTG ATC GGC CTT GCG CCC GCG GTC GCC TTT GCG
T V I G L A P A V A F A

TGC GAT CTG CCG CAA ACC CAC AGC CTG GGT AGC CGG CGA ACC TTG


ATG CTT CTG GCA CAG ATG CGG CGA ATC TCT CTT TTC TCT TGC TTA


AAG GAT CGA CAT GAC TTC GGT TTC CCG CAG GAG GAG TTC GGT AAC


CAG TTC CAA AAG GCT GAA ACC ATC CCG GTA TTG CAT GAG ATG ATC


CAG CAG ATC TTC AAC CTG TTC AGC ACT AAG GAC TCT TCT GCT GCT


TGG GAT GAG ACC CTG CTT GAC AAA TTC TAC ACT GAA CTG TAC CAG


CAG CTG AAC GAC CTG GAA GCC TGC GTG ATC CAG GGT GTG GGT GTG


ACT GAG ACT CCG CTG ATG AAG GAG GAC TCT ATT CTG GCT GTG CGA


AAA TAC TTC CAA CGG ATC ACT CTG TAT CTG AAA GAG AAG AAA TAC


AGC CCG TGC GCC TGG GAG GTT GTC CGA GCA GAA ATC ATG CGG TCT


TTC TCT TTG TCT ACC AAC TTG CAA GAA TCT TTA CGA AGC AAG GAA


TAA

TACGCGTACTAGTGAATTC-3'

The sequence as shown is divided into sub-paragraphs / regions which comprise:
1. the *gac* promoter and RBS (first paragraph, bps 257 to 465 of pMG414, see below),
2. the *gac* signal sequence coding region (second paragraph, bps 466 to 546 of pMG414, see below),
3. the synthetic gene for rhIFNα2b (third paragraph, bps 547 to 1044 of pMG414 (see below) - including the TAA stop codon), and
4. the 3' cloning linker (fourth paragraph, bps 1045 to 1063 of pMG414, see below).

On the pMG414 these four regions are directly joined to one another. They are separated in the figure for reasons of lucidity only.

The start (ATG) and the stop (TAA) codons of the open reading frame are shown in bold.

The first (TGC) an the last (GAA) codon of mature rhIFNα2b are underlined.

The restriction endonuclease sites used for cloning are boxed. These are:
- *Xba* I (TCTAGA) and *EcoR* I (GAATTC) for the introduction of the *gac* fragment (promoter, RBS, signal sequence, *Sac* II, *Mlu* I, *Spe* I sites)
- Sac II (CCGCGG) and Mlu I (ACGCGT) for the introduction of the rhIFNα2b PCR fragment (including four codons for the last four amino acids of the gac1ss, the 495 bp synthetic gene for mature rhIFNα2b, and the TAA(T) stop codon).

The gac promoter shows high constitutive / basal activity, the addition of a chemical inducer or a physical stimulus (change in culture conditions) is not required.

### 1.3 Cloning and establishment of the recombinant cell line

The expression plasmid pMG414 is introduced into the host strain ATCC PTA-3132 (=W3110 (ATCC 27325)) by electroporation. Electrocompetent cells are prepared according to a standard protocol, electroporation is carried out in 0.1 mm cuvettes at 1800 V using an Eppendorf Electroporator 2510.

After electroporation the reaction is suspended in liquid medium and plated onto agar plates containing tetracycline.

Starting point for selection of a suitable cell clone is a thus obtained transformation plate. Various clones from this plate are grown in liquid culture and cryopreserved as research cell banks. Their productivity is tested in shake flask experiments and compared. The best clone (E1/116) is used for further development.

The best clone may show good productivity but relatively poor growth. This poor growth can result from various factors, e.g. product toxicity to the host cell, metabolic burden due to product synthesis etc. The addition of glucose often brings some improvement because glucose downregulates (e.g. by catabolite repression) many promoters used for recombinant protein expression. Also, glucose has a general positive effect on the growth of *E. coli* because it can be directly introduced into the metabolism as a carbon source.

In the case of E1/116, a clear positive effect of glucose on growth is observed. The best results are achieved with glucose concentrations between 2 and 5 g/L. To adapt the cell line to cope with product formation and consequently to better growth in the absence of glucose, the strain is therefore grown in liquid medium in shake flasks for several passages ("shake flask cascade").

More specifically, a cryovial of E1/116 is thawed and the cell suspension streaked onto glucose free LB agar plates containing tetracycline. The plate is incubated at 37°C until the colonies reach a sufficient size for inoculating a liquid culture. Colonies are transferred from the plate into small shake flasks filled with 15 mL of glucose free LB broth containing tetracycline. The cultures are shaken at 37°C until they reach an optical density at 600 nm of above 0.5 (typically > 1.0). For this first round this takes up to 48 hours due to the poor growth characteristics of the original isolate.

The procedure described in the above paragraph is performed five consecutive times with the liquid culture of the previous round being streaked onto plates and the colonies from the plates serving to inoculate the next liquid culture. From each liquid culture optical density is determined and a sample was taken for determination of product titer using SDS-PAGE - Western Blot. Clones from the culture with the best combination of growth and productivity are the used to initiate the next round.

In the course of the different rounds of this culture cascade (i.e. multiple propagation and reisolation steps) the growth characteristics of the (sub)strain(s) gradually improve. By choosing the strain with the best combination of growth and productivity in each round, titers are also gradually increased. After the fifth round again single colonies are generated on LB agar plates containing tetracycline and used to inoculate a liquid culture containing tetracycline for the generation of a primary seed lot (PSL). The culture is grown at 37°C to an optical density of about 1.5, mixed with an equal amount of sterile 40% w/v glycerol, aliquoted into cryogenic vials and frozen at -80°C. This PSL is used as a starting point for the generation of the GMP cell banks (Master Cell Bank and Working Cell Bank) of the Interferon alpha 2b production strain. This reisolate is designated E1/116a. Reisolation processes like the one described above have proved to yield reproducible results.

E1/116a shows excellent growth characteristics in shake flasks and stirred bioreactors (fermenters). An inoculum suitable for starting a bioreactor can be grown in a shake flask starting from a Working Cell Bank vial in about 8 hours.

A Master Cell Bank is prepared under cGMP conditions from the primary seed lot described above. In brief, a PSL vial is thawed and plated onto tetracycline containing agar plates. A single colony is picked and used to inoculate the Master Cell Bank (MCB) shake flask culture (LB broth medium containing tetracycline). The cell supension from the logarithmic growth phase is mixed 1+1 with 40 % w/v Glycerol, aliquoted at 1.8 mL into cryogenic vials, sealed in cryogenic tubing, and frozen in the liquid phase of a liquid nitrogen tank.

The Working Cell Bank is generated in the same way as the Master Cell Bank except that the shake flask culture is inoculated with cell suspension from a thawed MCB vial.

### Example 2: Fermentation process for production of recombinant human interferon a 2B (rhIFNα2B)

The fermentation process is started by growing the strain E.coli K-12 W3110 obtainable from the Working Cell Bank as described above in shake flask cultures in Luria Bertani (LB-) medium at 37°C with the addition of the antibiotic tetracyclin hydrochloride to avoid growth of non-plasmid carrying cells.

The shake flask culture is then used to inoculate the seed culture (= pre-culture) medium (inoculum size = 0,4%). The medium for this pre-culture cultivation is based on deionized water containing glucose as a sole carbon source and yeast autolysate as a complex nitrogen source. In addition, anorganic salts like KH₂PO₄, K₂HPO₄, (NH₄)₂SO₄ and MgSO₄.7H₂O are added to the medium. As an antifoam agent polypropylene glycole 2000 (PPG2000) is used. In particular, the pre-culture medium has the following composition:

### Pre-culture Basal Medium:

| **Component** | **Amount** |
|---|---|
| De-ionized water (WBI) | 30 l |
| Yeast autolysate, KAT, Ohly | 21.7 g/l |
| Glucose Monohydrate, pure | 25.0 g/l |
| Ammonium Sulfate, p.a. | 1.0 g/l |
| Potassium Phosphate Monobasic, p.a | 1.5 g/l |
| Potassium Phosphate Dibasic, anhydrous, pure | 3.0 g/l |
| Magnesium Sulfate Heptahydrate, p.a. | 0.5 g/l |
| Polypropylene Glycole 2000 | 0.5 ml/l |

These media components are sterilized together for 20 minutes at 121 °C. After cooling of the basal medium, an aliquot of a 5 g/L sterile stock-solution of the antibiotic Tetracycline Hydrochloride is added to the basal medium (sterilization is performed by filtration (0.22 µm filter)).

### Stock-solution Tetracycline Hydrochloride (5 g/L):

| **Component** | **Amount** |
|---|---|
| Tetracycline Hydrochloride cryst., Ph.Eur. | 15 mg/l |
| De-ionized water (WBI) | |

The cultivation-time for seed culture is about 16 hours. During cultivation of the seed culture pH-value is controlled to a set-point of 7,0 ± 0,2 with sulfuric acid and sodium hydroxide or concentrated ammonia solution. Concentration of dissolved oxygen is kept at levels higher than 20% of saturation by increasing the stirrer speed. Stirrer speed at the beginning of the cultivation is set to 300 rpm, back-pressure in the vessel to 0,3 bar and aeration rate is controlled to 30 Umin (equivalent to "1 vvm"). Temperature is kept constantly at 37°C during cultivation. As a transfer criterion of broth to the main stage of the fermentation process, an increase of the dissolved oxygen concentration after consumption of the carbon source is used.

For main culture cultivation a medium based on deionized water, glucose as a carbon source and yeast autolysate as a complex nitrogen source is used. Besides the addition of the anorganic salts (NH₄)₂SO₄, CaCl₂.2H₂O and MgSO₄.7H₂O, PPG 2000 is used as an antifoam agent. The initial glucose is sterilized separatly and added to the sterile rest of the medium. Inoculum size to the main fermenter medium was in a range between 0,75 and 3%. In particular, the main culture medium has the following composition:

### Main Culture Basal Medium:

| **Component** | **Amount** |
|---|---|
| Deionized water (WBI) | 60 l |
| Yeast autolysate, KAT, Ohly | 43.5 g/l |
| Ammonium Sulfate, p.a. | 1.0 g/l |
| Calcium Chloride Dihydrate cryst., p.a. | 0.3 g/l |
| Magnesium Sulfate Heptahydrate, p.a. | 1.0 g/l |
| Polypropylene Glycole 2000 | 0.5 ml/l |

These media components are sterilized together for 20 minutes at 121 °C. After cooling, an aliquot of a 800 g/L separately heat-sterilized glucose stock-solution is added to the main culture basal medium (sterilization is performed for more than 30 minutes at 120 °C).

### Glucose Stock-solution, 800 g/l:

| **Component** | **Amount** |
|---|---|
| Glucose Syrup | 12.5 ml/l |
| De-ionized water (WBI) | |

The most important point during this cultivation is the necessity of a complete consumption of the initial glucose present in the medium. This leads to a sharp increase of dissolved oxygen concentration after about 9 hours of growth. By starting glucose feeding before total consumption of the initial glucose, no product formation is observed. Glucose limitation controlled by the feeding of the glucose-solution at a constant rate is therefore very important. The temperature during cultivation is controlled to a constant value of about 28°C. The initial stirrer speed is set to 300 rpm, the aeration rate is controlled to 100 Umin (equivalent to "1 vvm") and the back-pressure in the vessel is set to 0,3 bar. The pH-value is controlled to 7,1 ± 0,3 with sulfuric acid and sodium hydroxide or concentrated ammonia solution. A peak of the pH-value up to 8.0 after consumption of the initially supplied glucose is acceptable.

The concentration of the dissolved oxygen is controlled to values higher than 20% of saturation. Dependent on the oxygen transfer capacity of the bioreactor DO-concentration is kept at levels higher than 20% of saturation, preferably between about 40 % and 100% of saturation, by first increasing the stirrer speed to a maximum value. If this is not sufficient, first aeration rate and after that back-pressure is increased, respectively. After a cultivation time between 48 and 192 hours (linear increase of product formation is observed with cultivation time) the culture is harvested and cooled to 15 ± 5 °C and conditioned for downstream processing by the addition of sucrose/EDTA to the cooled broth.

The results of a fermentation batch is analysed based on the Westernblot technique or on HPLC-measurements after laboratory or pilot plant periplasmatic extraction of the product.

### Example 3: Cell disruption and extraction

A fermentation broth containing host cells comprising the expressed interferon alpha 2B in the periplasmic space is adjusted with sulfuric acid to pH of 5,0 ± 0,1 immediately after the fermentation and cooled down to 4°C ± 2 °C. The low pH and the low temperature help to inactivate endogenous proteases.

The fermentation broth is adjusted to 10°C to 20°C, then without any concentration or washing of the cells, solid or liquid sucrose (200g sucrose/kg fermentation broth) and EDTA (concentration 10mM) are added and the pH adjusted to 8.0. After a selective one-step cell permeation protocol using osmotic shock (1+3 dilutions) with cooled water, whereby the fermentation broth comprising sucrose and EDTA is poured or pumped into the cooled (temperature about 4°C) water, the released periplasmic extract is clarified. Polyethyleneimine is added to a final concentration of 0,05 % and the pH is adjusted to about 7,5 with acetic acid. After 15 to 45 minutes cell debris and DNA flocculate, leaving a clear crude extract containing interferon which may be subject to centrifugation to improve clarity.

This procedure leads to a clear periplasmic extract comprising the desired interferon alpha 2B in high yield with a purity of > 20 % with respect to the total protein content. Polyethyleneimine helps to separate the cell debris from the soluble protein extract leading to a very pure interferon solution.

### Example 4: Chromatographic purification of recombinant human interferon α 2B (rhIFNα2B)

### 4.1 Capture by Cation Exchange Chromatography (CEX)

After pH adjustment to 4.8 - 5.2 with acetic acid and a filtration step using a 0.3 micron filter, the crude extract of Example 3 is applied to the CEX column (S ceramic HyperD F (Biosepra)). After a washing step with an equilibration buffer (20mM sodium acetate and 70mM NaCl at pH 5.0) the interferon is eluted with a step gradient at 175mM NaCl. The fraction collected is immediately processed by the process step of Example 4.2.

### 4.2 Anion Exchange (AEX) Chromatography

The fraction from Example 4.1 is adjusted to a pH of 7.3 to 7.7 with sodium hydroxide, diluted and purified with water to a conductivity of 3.5 to 4.5 mS/cm and applied to the AEX column (Q ceramic HyperD F (Biosepra)). After washing, the interferon is eluted with a linear salt gradient (0-300mM NaCl) at about 150mM NaCl. Fractions are collected that have a purity of greater than or equal to 90 area % according to IPC reversed-phase HPLC and used directly in the next step (see Example 4.3).

### 4.3 Hydrophobic Interaction Chromatography (HIC)

The fraction of Example 4.2 is diluted (1:1) with a stock solution of sodium sulphate (0.5% sodium sulphate), adjusted to pH 7.3 to 7.7 with NaOH or HCl and applied to the HIC column (Source 15PHE (Pharmacia). After washing, the interferon fraction of Example 3 is eluted with a linear sodium sulphate concentration (800 - 0 mM sodium sulphate) at about 400mM sodium sulphate. The fractions collected that have a purity of greater than or equal to 93 area % and no impurity greater than or equal to 3% according to IPC reversed-phase HPLC are used directly in the next purification step.

### 4.4 Cation Exchange Chromatography (CEX)

The collected fractions of Example 4 are diluted with water to a final conductivity of 7.5 to 8.5 mS/cm, adjusted to pH 4.3 to 4.7 with 99 to 100% acetic acid and applied to the CEX column (Toyopearl SP-650 S (TosoHaas)). After a washing step, the interferon is eluted in a linear NaCl gradient (0 - 300 mM NaCl) at about 250mM NaCl. The fractions are collected that have a purity of greater than or equal to 95 area % and no impurity greater than or equal to 3% according to IPC reversed-phase HPLC and are used directly in the next purification step.

### 4.5 Size Exclusion Chromatography

The last purification step is a gel filtration step to remove dimers and other aggregates and to perform a buffer exchange for the final formulation. The Superdex 75 pg used in this step shows a good resolution even at a high load volume (5 % - 15 %). The SEC is performed in 25mM sodium phosphate and 130mM NaCl + 0.3mM EDTA at a pH of about 7.3 to 7.7.

The fractions with the highest purity (> 95 % main peak in RP-HPLC and no side peak > 3 %) are pooled to give the final bulk solution comprising the desired recombinant human interferon α 2B in pure form in a high yield.

### Deposition of Microorganisms:

E. coli strain W3110 (ATCC 27325), as used herein, has been deposited with the American Type Culture Collection (ATCC), 10801 University Boulevard, Manassas, VA 20110-2209, USA on February 28, 2001, under the Designation No. PTA-3132.

## Claims

1. A process for the preparation of a recombinant polypeptide of interest, comprising
(i) fermentation of a prokaryotic host cell comprising a periplasm and being transformed with a recombinant expression system capable of bringing about secretion of a polypeptide of interest into the periplasm of said host cell, wherein said fermentation is performed in a fermentation medium under conditions such that the polypeptide of interest is secreted into the periplasm of the host cell, and
(ii) extraction of the polypeptide of interest from the periplasm by applying an osmotic shock to the host cells contained in the fermentation medium.
wherein said osmotic shock is performed by adding sucrose directly to the fermentation medium and subsequent dilution with H₂O such that the outer cell membrane of the host cell is sufficiently disrupted as to release the contents of its periplasm into the fermentation medium.

2. The process according to claim 1, wherein the concentration of the sucrose in the fermentation medium when starting the dilution is about 20% weight/volume.

3. The process according to claim 2, wherein the dilution factor of the sucrose-containing fermentation broth with H₂O is at least 3 times.

4. The process according to claim 1, wherein said prokaryotic host cell is a Gram-negative bacterium.

5. The process according to claim 4, wherein said Gram-negative bacterium is selected from the group consisting of Escherichia coli, Pseudomonas sp., Enterobacter sp., Campylobacter sp. and Vitreoscilla sp.

6. The process according to claim 5, wherein the Gram-negative bacterium is E. coli.

7. The process according to claim 1, wherein the polypeptide of interest is selected from the group consisting of an interferon, an interleukin, a growth hormone, a growth factor, a cytokine, an enzyme, an enzyme inhibitor, an antibody and an antibody fragment.

8. The process according to claim 1, wherein the polypeptide of interest is an interferon alpha 2.

9. The process according to claim 8, wherein the interferon alpha 2 is selected from the group consisting of interferon alpha 2A and interferon alpha 2B.

## Patentansprüche

1. Verfahren zur Reinigung eines gewünschten rekombinanten Polypeptids, umfassend
(i) Fermentation einer prokaryotischen Wirtszelle umfassend ein Periplasma und transformiert mit einem rekombinanten Expressionssystem, welches in der Lage ist, die Sekretion eines gewünschten Polypeptids in das Periplasma dieser Wirtszelle zu verursachen, worin diese Fermentation in einem Fermentationsmedium unter solchen Bedingungen durchgeführt wird, dass das gewünschte Polypeptid in das Periplasma der Wirtszelle sekretiert wird, und
(ii) Extraktion des gewünschten Polypeptids aus dem Periplasma durch Anwendung eines osmotischen Schocks auf die Wiztszellen, welche im Fermentationsmedium enthalten sind, worin dieser osmotische Schock durch direkte Zugabe von Sucrose zu dem Fermentationsmedium und anschließende Verdünnung mit H₂O durchgeführt wird, so dass die äußere Zellmembran der Wirtszelle ausreichend zerstört wird, um den Inhalt ihres Periplasmas in das Fermentationsmedium freizusetzen.

2. Verfahren nach Anspruch 1, worin: die die Konzentration der Sucrose im Fermentationsmedium zu Beginn der Verdünnung bei etwa 20% Gewicht/Volumen liegt.

3. Verfahren nach Anspruch 2, worin der Verdünnungsfaktor der Sucrose-enthaltenden Fermentationsnährlösung mit H₂O mindestens 3fach ist.

4. Verfahren nach Anspruch 1, worin die prokaryotische Wirtszelle ein Gram-negatives Bakterium ist.

5. Verfahren nach Anspruch 4, worin das Gram-negative Bakterium ausgewählt ist aus der Gruppe bestehend aus Escherichia coli, Pseudomonas sp., Enterobacter sp., Campylobacter sp. und Vitreoscilla sp.

6. Verfahren nach Anspruch 5, worin das Gram-negative Bakterium E. coli ist.

7. Verfahren nach Anspruch 1, worin das gewünschte Polypeptid ausgewählt ist aus der Gruppe bestehend aus einem Interferon, einem Interleukin, einem Wachstumshormon, einem Wachstumsfaktor, einem Zytokin, einem Enzym, einem Enzyminhibitor, einem Antikörper und einem Antikörperfragment.

8. Verfahren nach Anspruch 1, worin das gewünschte Polypeptid ein Interferon alpha 2 ist.

9. Verfahren nach Anspruch 8, worin das Interferon alpha 2 ausgewählt ist aus der Gruppe bestehend aus Interferon alpha 2A und Interferon alpha 2B.

## Revendications

1. Procédé de préparation d'un polypeptide recombinant d'intérêt, comprenant
(i) la fermentation d'une cellule hôte procaryote comprenant un périplasme et étant transformée avec un système d'expression recombinant capable de réaliser la sécrétion d'un polypeptide d'intérêt dans le périplasme de ladite cellule hôte, où ladite fermentation est réalisée dans un milieu de fermentation dans des conditions telles que le polypeptide d'intérêt est sécrété dans le périplasme de la cellule hôte, et
(ii) l'extraction du polypeptide d'intérêt à partir du périplasme en appliquant un choc osmotique aux cellules hôtes contenues dans le milieu de fermentation,
où ledit choc osmotique est réalisé en ajoutant du saccharose directement au milieu de fermentation et ensuite en diluant avec H₂O de telle manière que la membrane cellulaire externe de la cellule hôte est suffisamment rompue pour libérer le contenu de son périplasme dans le milieu de fermentation.

2. Procédé selon la revendication 1, dans lequel la concentration du saccharose dans le milieu de fermentation au départ de la dilution est d'environ 20 % en poids/volume.

3. Procédé selon la revendication 2, dans lequel le facteur de dilution du bouillon de fermentation contenant le saccharose avec H₂O est d'au moins 3 fois.

4. Procédé selon la revendication 1, dans lequel ladite cellule hôte procaryote est une bactérie Gram négatif.

5. Procédé selon la revendication 4, dans lequel ladite bactérie Gram négatif est choisie dans le groupe constitué par *Escherichia coli, Pseudomonas sp*, *Enterobacter sp, Campylobacter sp* et *Vitreoscilla sp.*

6. Procédé selon la revendication 5, dans lequel la bactérie Gram négatif est *E. coli.*

7. Procédé selon la revendication 1, dans lequel le polypeptide d'intérêt est choisi dans le groupe constitué par un interféron, une interleukine, une hormone de croissance, un facteur de croissance, une cytokine, une enzyme, un inhibiteur enzymatique, un anticorps et un fragment d'anticorps.

8. Procédé selon la revendication 1, dans lequel le polypeptide d'intérêt est un interféron alpha 2.

9. Procédé selon la revendication 8, dans lequel l'interféron alpha 2 est choisi dans le groupe constitué par l'interféron alpha 2A et l'interféron alpha 2B.
